# EUROPEAN PATENT APPLICATION

(11) **EP 1 923 403 A1**
(43) Date of publication of application: **21.05.2008**
(21) Application number: 06077029.4
(22) Date of filing: 15.11.2006
(51) Int. Cl.: C08C 1/04, C08J 5/02, C08L 7/02, A61B 19/04

(54) **Latex products such as gloves**

(71) Applicant: Nederlandse Organisatie voor toegepast- natuurwetenschappelijk onderzoek TNO, 2628 VK The Hague (NL)
(72) Inventor: Slaghek, Theodoor Maximiliaan, 3042 HT Rotterdam (NL); Timmermans, Johannes Wilhelmus, 6714 GG Ede (NL); Naus, Johannes Hubertus, 5216 GA S'Hertogenbosch (NL); Jetten, Jan Matthijs, 3701 JL Zeist (NL); Havinga, Jakob Sikkes, 2613 BL Delft (NL)
(74) Representative: van Loon, C.J.J.

(57) **Abstract**

Method for the manufacture of latex products, like latex gloves etc., from a latex starting material which is mixed with particles which are capable to absorb and/or immobilize proteins present in the latex material, like charged crosslinked biopolymer particles. In addition, the latex products may comprise a powder layer of said particles, applied on the surface of the latex product.

## Description

### Field

The present invention is directed to a method for the manufacture of products from a latex starting material, especially - however not exclusively - latex products which are intended for use in contact with the human body, like latex gloves, condoms, catheters, stomach balloons etc.

### Background

The invention concerns the avoiding of the development of protein allergy, which is caused by the use of natural rubber latex, hereinafter simply called latex. This phenomenon has start in the beginning of the eighties of the past century and expresses itself in three different reactions, namely:
- skin irritation: this mostly disappears when no use is made of latex during some time;
- delayed hypersensitivity: this means that frequent use of natural rubber leads, in the long run, to an ever increasing allergic reaction,
- direct hypersensitivity: this means that nearly immediately an allergic reaction occurs when used.

Allergic reactions have increased considerably since the use of rubber gloves in the medical world. Latex gloves have a number of advantages compared to gloves made of synthetic rubbers and other synthetics. Especially the supple fit, the good grip and the high level of tear strength, important during surgeries, are the most prominent advantages.

The allergic reaction develops because compounds, which are present in the latex by nature, slowly leak from the matrix and get in touch with the skin. These compounds are particularly proteins and protein fragments, which easily enter into the skin and can initiate an allergic reaction in that way. Studies are known which give evidence that the sweat can act as a solvent for the allergens and in that way promotes the absorption by the skin. Besides, the use of maize [corn] starch for powdering the gloves is an additional source of allergenic concentration because the starch is capable to bind the allergenic compound and the allergens can be spread through the air while pulling off the gloves.

During the last decade various measurements were successful in diminishing the content of extractable proteins in latex gloves. During the production process the products are abundantly rinsed with water, as a result of which part of the proteins are washed out. Additionally, latex has come on the market that has been deproteinized with the help of enzymes; this latex, however, is expensive. By using gloves with low protein concentrations allergy is not built up any longer and in consequence, new cases of latex sensitivity can be avoided. A problem is formed by the persons already sensitized who also react on low concentrations. Those people form a high-risk group and therefore latex is more and more avoided, especially in hospitals.

Because of latex allergy new alternative materials have come on the market since the mid-nineties of the past century. Especially synthetic rubbers as nitrile rubber and isoprene rubber and plastics as polyvinylchloride and polyurethane have been used as a basis. It has be proven that characteristics as comfort, grip, fit and strength are often less favourable than latex and that the gloves are quite more expensive. Therefore there seems to be a positive balance for natural product latex. Known methods are directed towards a process for decreasing the quantity of protein (US 5908893 and US 6754281), the use of enzymes (proteases), and anionic surfactants (US 6204358), as well as methods in order to identify allergenic proteins (US6759517).

### Summary

The invention is based on the idea that when it might be possible to bind proteins and protein fragments and to immobilize them, it would be possible to prevent the penetration of such proteins into the skin, thus improving the allergenic characteristics and thus the acceptance of latex gloves.

The invention thus comprises a method for the manufacture of latex based products, like e.g latex gloves, etc., from a latex comprising starting material, the method comprising a process step wherein said latex starting material is mixed with particles which are capable to absorb or immobilize (or both) proteins present in the latex material. The particles to be mixed may be charged crosslinked particles, e.g. biopolymer particles.

Besides the method for the manufacture of latex products, also latex products which are manufactured by the method according to the invention, are deemed to be part of the invention.

So, in brief, the improved method comprises mixing into the latex of particles which are capable of absorbing or immobilizing (or both) the proteins in the latex, resulting in that the proteins do not longer leave the finished product and thus cause no more allergic reactions.

Additionally, such (absorbing/immobilizing) particles may also be applied - in the form of a powder "layer" - on the surface of the gloves. The particles can absorb the sweat getting from the skin and thus increase the comfort. The powder particles are capable to bind the proteins that may still leak from the latex matrix and in that way make that the allergenic proteins are being immobilized and in that way can cause no more allergenic reaction.

One preferred class of polymers to be used in the present invention may be derived from any carbohydrate polymer comprising a primary alcohol group. Such carbohydrate polymers include non-reducing disaccharides and oligosaccharides, such as sucrose, raffinose, trehalose and similar oligosaccharides, and polysaccharides, which are 1,2-, 1,4- or 1,6-linked.

Examples include α-1,4-glucans (the "starch family"), β-1,4-glucans (cellulose), glucomannans and galactomannans (guar and locust bean gum), (arabino) xylans (hemicellulose) and β-2,1 and β-2,6-fructans (inulin and levan). The starch-type carbohydrates, cellulose and fructans are preferred carbohydrate polymers.

Modifications of starch and other carbohydrate polymers may also be used as starting materials, and comprise partially hydrolysed products, as well as physical and chemical modifications, including kationisation, hydroxyalkyl, carboxyalkyl and similar derivatives, as well as uronic acid analogues.

Suitable examples of carboxylated carbohydrates include glucans, especially α-glucans, in which the anhydroglucose units are linked through 1,3 - 1,4- and/or 1,6-linkages. These glucans include starch, starch components or starch variants such as native starch from any biological source including potato, manioc, tapioca, wheat, maize, rice, banana, quinoa and the like, as well as from genetically modified plants and micro organisms, and starch fractions, amylose, amylopectin, high-amylose starch etc. They also include other branched and non-branched 1,3-, 1,4- and 1,6-glucans (beta-glucans), whether from plant or from microbial origin, such as those described in US 6,486,314, US 6,465,203 and WO 03/008618. Other carboxylated carbohydrates having the required molecular weight and solubility, such as glucomannans, galactomannans, xyloglucans etc. may also be used as suitable absorption/immobilization agents to be mixed, according the present invention, in the latex starting material and/or applied at the surface(s) of the relevant end products, e.g. gloves etc.

### Exemplary Embodiments

Below, some examples will be given for the preparation of agents which, according the present invention, are suitable to be mixed in the latex starting material and/or applied at the surface(s) of the relevant end products.

### Example A (Synthesis of the polymer matrix with one type of biopolymer):

A solution of 58 mg of NaOH in 90 ml of water was cooled to 0°C. To this was added 0.4 ml of divinyl sulfone. Slowly added to this were 15 grams of C-6 oxidized starch (degree of oxidation 30%). The solution changed overnight into a transparent colorless gel. This gel was pressed through a sieve of ca. 1 mm² mesh size, after which 0.5 liter of water was stirred through the gel, which was absorbed directly. After this, the gel was precipitated with 1 liter of ethanol and then washed twice with ethanol and once with acetone, after which the gel was cried in air.

### Example B (Crosslinking oxidized starch with divinyl sulfone):

To a solution of 52 mg of NaOH in 90 ml of water, 10 grams of C6-oxidized starch (degree of oxidation 30%) were added with stirring. Next, with the aid of an ice bath, the temperature was adjusted to 0 - 5°C. After this, 800 µl of divinyl sulfone were added with stirring. After 5 minutes of stirring, the solution was stored overnight at room temperature. The transparent and virtually colorless gel obtained was pressed through a sieve (1 mm²) and suspended in 0.5 liter of water, whereby the gel swelled strongly. Next, the gel was precipitated by adding 1 liter of ethanol. After that, the precipitate was washed twice with ethanol, once with acetone and finally dried in air. The yield was 9.0 grams with a free swell of 49.

### Example C (Crosslinking cationic starch with epichlorohydrin):

To a solution of 2.4 grams of NaOH in 480 ml of water, 120 grams of Paselli S.A.-2, and then 73 ml of glycidyltrimethylammonium chloride (70% in water) were added. After this, the reaction mixture was stirred for 2 hours at 60 °C. After cooling, to 100 ml of this reaction mixture, 1 gram of NaOH and 3 ml of epichlorohydrin were added. The solution was stirred for 15 minutes and then stored for 3 days at 37 °C. The gel obtained was pressed through a sieve (1 mm²) and washed 10 times with 1 liter of water, 3 times with 1 liter of ethanol and 3 times with acetone. The precipitate was dried in air. The yield was 18.05 grams of powder with a swelling capacity of 58 in water.

### Example D (Crosslinking cationic starch with epichlorohydrin):

To a solution of 2.4 grams of NaOH in 480 ml of water, 120 grams of Paselli S.A.-2, and then 146 ml of glycidyltrimethylammonium chloride (70% in water) were added. After this, the reaction mixture was stirred for 2 hours at 60 °C. After cooling, to 100 ml of this reaction mixture, 1.6 grams of NaOH and 5 ml of epichlorohydrin were added. The solution was stirred for 15 minutes and then stored for 3 days at 37 °C. The gel obtained was pressed through a sieve (1 mm²) and washed 10 times with 1 liter of water, 3 times with 1 liter of ethanol and 3 times with acetone. The precipitate was dried in air. The yield was 22 grams of powder with a swelling capacity of 32 in water.

### Example E (Crosslinking of carboxymethylcellulose with epichlorohydrin):

A solution of 1.5 grams of NaOH in 160 ml of water was vigorously stirred while 15 grams of carboxymethylcellulose (medium viscosity, Sigma) and then 2.5 ml of epichlorohydrin were added. After 15 minutes of stirring at room temperature, the solution was stored for 3 days at a temperature of 37°C. After cooling to room temperature, the very tough and transparent gel was cut into pieces of about 1 cm. These gel pieces were preserved overnight in 3 liters of water. Nearly all of the water was thereby absorbed and colorless, transparent lumps were obtained that were hard and brittle. These were pressed through a sieve (1 mm²) and washed 10 times with 1 liter of water, 3 times with 1 liter of ethanol and 3 times with acetone and then dried in air. This resulted in 9.8 grams of dry gel having a free swell of 228 grams of water per gram of gel.

So, in the foregoing a number of examples are given of agents which are suitable to be mixed, e.g. by means of well known techniques like stirring, extrusion etc., in latex material to be used to manufacture latex gloves etc. showing improved charactistics w.r.t. latex allergy. Moreover, those agents will be fit to be supplied to the surface - e.g. the gloves' inner surface - of the end products.

For the sake of intelligibility, where needed, reference could be made to applicant's preceding patent disclosures WO2005032252 (examples 1, 5, 9, 10 and 11), WO2005090462 and WO2005080499.

## Claims

1. Method for the manufacture of products from a latex starting material, the method comprising a process step wherein said latex starting material is mixed with particles which are capable to absorb and/or immobilize proteins present in the latex material.

2. Method according to claim 1, wherein said particles comprise charged crosslinked particles.

3. Method according to claims 1 or 2, wherein said particles comprise biopolymer particles.

4. Method according to claim 3, wherein said biopolymer particles comprise starch or a derivative of starch.

5. Method according to claim 3, wherein said biopolymer particles comprise cellulose or a derivative of cellulose.

6. Latex product, manufactured from a latex starting material, comprising particles which are capable to absorb and/or immobilize proteins present in the latex material.

7. Latex product according to claim 6, wherein said particles comprise charged crosslinked particles.

8. Latex product according to claims 6 or 8, wherein said particles comprise biopolymer particles.

9. Latex product according to claims 6, 7 or 8, comprising a powder layer of said particles, applied on the surface of the latex product.

10. Latex product according to claim 8 or 9, wherein said particles comprise starch or a derivative of starch.

11. Latex product according to claim 8 or 9, wherein said particles comprise cellulose or a derivative of cellulose.
